# DEMANDE DE BREVET EUROPEEN

(11) **EP 4 817 592 A1**
(43) Date de publication de la demande: **07.10.2026**
(21) Numéro de dépôt: 26165398.4
(22) Date de dépôt: 17.03.2026
(51) Int. Cl.: A61K 8/25, A61K 8/60, A61P 17/08, A61P 17/10, A61Q 5/00, A61Q 7/00, A61Q 19/00, A61Q 19/08, A61K 8/9789

(54) **COMPLEXE ASSOCIANT UNE BIOMOLÉCULE AZOTÉE HÉTÉROCYCLIQUE AVEC UN ACIDE SILICIQUE, ET APPLICATIONS**

(30) Priorité: 01.04.2025 FR 2503392
(71) Demandeur: Exsymol, 98000 Monaco (MC)
(72) Inventeur: CAPPONI, Marco, 18100 IMPERIA (IT)
(74) Mandataire: Murgitroyd & Company

(57) **Abrégé**

La présente invention concerne de nouveaux complexes formés entre a) une biomolécule azotée hétérocyclique et b) un acide silicique, les compositions les comprenant, ainsi que leurs applications.

## Description

### Domaine de l'invention

La présente invention concerne de nouveaux complexes associant une biomolécule azotée hétérocyclique avec un acide silicique, les compositions les comprenant, ainsi que leurs applications.

### Etat de la technique

Les acides nucléiques, y compris les deux formes majeures que sont l'acide désoxyribonucléique (ADN) et l'acide ribonucléique (ARN), sont des macromolécules naturelles présentes universellement dans tout organisme vivant. En plus de leur singularité largement étayée d'être le matériel héréditaire à l'origine du stockage, codage et transfert de l'information génétique à d'autres composants cellulaires, leur rôle est également fondamental chez l'Homme à travers leur implication dans de très nombreux processus biologiques de l'organisme : catalyse de certaines réactions biochimiques, régulation de certaines activités dans les cellules, etc. (Wang F. et al., Biosensors, 2022, vol.12, pp.1-35 et références citées). Tel l'énoncé figurant dans le guide en ligne des ingrédients cosmétiques destinés à des formulations de beauté de premier ordre, les acides nucléiques représentent aujourd'hui un groupe de molécules bioactives et d'ingrédients qualifiés "de pointe" car innovants, scientifiquement prouvés, efficaces et incorporés le plus souvent dans des produits de soins exclusifs (https://inci.guide/nucleic-acids). De ce fait, en cosmétique, en dermatologie ou encore dans les domaines de la médecine esthétique ou régénérative, ces acides nucléiques, ainsi que leurs fragments tels que les polynucléotides et polydésoxyribonucléotides (biopolymères linéaires souvent décrits, par commodité, à l'aide des abréviations respectives et communes "PN" et "PDRN"), voire même leurs simples unités monomériques basiques constitutives, ont vu ces dernières années leur exploitation afficher un potentiel grandissant et un essor marquant lorsqu'il s'agit de viser le renouvellement cutané et la bio-restructuration de la peau (Zakrewsky M. et al., J. Control Release, 2015, vol.219, pp.445-456). Ainsi, à titre de première illustration quant à l'action et intérêt pour la peau des précités "acides nucléiques et fragments résultants", un article de la présente année fait état, pour les susnommés PNs, de résultats prometteurs dans la réduction en profondeur des rides, dans leur capacité à améliorer la texture de la peau, etc., et au-delà, en conclusion, d'une intervention cosmétique jugée efficace et bien tolérée (Lampridou S. et al. J. Cosmet. Dermatol., 2025, vol.24, e16721). Quelques mois auparavant, une autre étude menée sur ces mêmes PNs rapportait, elle, des améliorations significatives dans l'élasticité et l'hydratation de la peau (Lee K.W.A. et al., Int. J. Mol. Sc., 2024, vol.25, 8224). Quant aux susnommés PDRNs, à comprendre par des fragments naturels d'ADN de bas poids moléculaire compris entre 50 et 1 500 kDa (Marques C. et al., Biomolecules, 2025, vol.15, 148), l'état de la technique leur confère un vaste ensemble de bénéfices cutanés, allant de la promotion de l'activité cellulaire et la synthèse de collagène au traitement de l'hyperpigmentation, en passant par la revitalisation de la peau, l'angiogenèse, etc. (Khan A. et al., Chinese J. Plastic Rec. Sur., 2022, vol.4, pp. 187-193), ce qui a d'ailleurs permis à cette même équipe d'auteurs de retenir un titre suggestif pour leur article avec : « *Polydeoxyribonucleotide : a promising skin anti-aging agent* »*.* Corrélativement au constat ci-dessus, il est également reconnu à ces mêmes PDRNs une capacité à stimuler la néocollagénèse de peaux photo-vieillies, mais aussi des potentialités dans le blanchiment de la peau en raison de propriétés dépigmentantes (Noh T.K. et al., Int. J. mol. Sc., 2016, vol.17, 1448). Par ailleurs, à titre de seconde illustration, dirigée celle-ci autour de l'action et l'intérêt cutanés des précitées "unités monomériques basiques constitutives des acides nucléiques", en d'autres termes les cinq bases azotées puriques et pyrimidiques que sont l'adénine, la guanine, la cytosine, la thymine, et l'uracile, l'état de la technique affiche par exemple, tel pour l'adénine, base nucléique purique, une capacité à retarder la sénescence de cellules situées dans le derme papillaire, zone fine située au niveau de la jonction dermo-épidermique de la peau (Cheng Y-F. et al., Exp. Dermatol., 2016, vol.25, pp.151-164). Une même adénine, certes fonctionnalisée avec le composé dénommé N⁶-(4-hydroxybenzyl)adénine riboside, démontre, elle, sur un modèle murin de vieillissement, des vertus contre la sénescence cellulaire induite par des typologies distinctes de stress oxydatif, en conséquence une capacité à pouvoir être un puissant agent pour prévenir la neurodégénérescence et la neuro-inflammation liées au vieillissement (Hsu W.H. et al., Aging, 2020, vol.12(11), pp.10556-10577). Une autre adénine ribosylée s'affiche, d'une part, dans une poudre composite comprenant également du niacinamide et une cyclodextrine, dans le cadre de la prévention de la chute des cheveux (demande de brevet WO2022/239914 A1) et, d'autre part, à titre d'agent "fonctionnel" au sein d'une composition cosmétique destinée à corriger plusieurs désordres cutanés (demande de brevet KR20160055597 A). De son côté, à des fins applicatives en cosmétique et en dermatologie, la guanine, autre base nucléique purique, est considérée, lorsqu'elle se trouve associée à un aldopentose, comme un ingrédient actif utile dans le maintien de l'homéostasie de la peau, en particulier dans la prévention et/ou le traitement des irritations cutanées (brevet FR2975595). Il peut également être souligné que l'acide nucléique sous sa forme prééminente (l'ADN) se compose, parmi la liste des cinq susnommées bases azotées, de quatre d'entre elles, et que chacune de ces quatre nucléobases (adénine, guanine, cytosine et thymine) se trouve répertoriée dans le dictionnaire "International Cosmetic Ingredient Dictionary and Handbook" publié par le "Personal Care Products Council (PCPC)" de l'Association cosmétique américaine, en d'autres termes le référentiel utilisé mondialement par l'industrie cosmétique pour standardiser et normaliser la dénomination de tout ingrédient présent dans les formules commercialisées de produits de soin. En tout état de cause et à ce niveau de description de la présente invention, il est bon de retenir que l'exposé des bénéfices cutanés liés à l'exploitation des acides nucléiques et dérivés en tant qu'ingrédient actif et fonctionnel sur la peau est aujourd'hui dense et prometteur, avec notamment la dernière décennie qui a vu publier un grand nombre d'études sur le sujet. Au sens de la présente demande, il convient d'ailleurs d'appréhender les susnommés « acides nucléiques et dérivés » en adoptant une approche holistique, c'est-à-dire englobant tant une macromolécule (polymère) organique constituée d'une succession de nucléotides que ses unités monomériques constitutives (ces dernières pouvant être libres ou fonctionnalisées par une liaison N-osidique). Il reste que, malgré ce travail considérable déployé pour mieux appréhender les cibles cutanées et mécanismes d'action sur la peau de ces acides nucléiques et dérivés, plusieurs limites et obstacles subsistent toujours à leur emploi et se doivent d'être, au cas par cas, considérés pour une utilisation escomptée optimale en cosmétique et dermocosmétique. A cet égard, il est possible de citer un problème de stabilité, notamment en application topique cutanée, de ces acides nucléiques et dérivés, en particulier en raison de facteurs environnementaux (rayonnement ultraviolet, chaleur, humidité), biologiques (nucléases et enzymes naturelles de la peau), chimiques (pollution, variations de pH, interaction avec d'autres ingrédients), tous susceptibles ainsi d'entraîner leur dégradation et, par voie de conséquence, une perte d'efficacité (Ingle R.G. et al., Pharmaceutics, 2023, vol.15, 1158 ; Probst J. et al., Genetic Vaccines and Therapy, 2006, vol.4(4), pp.1-9). Tel qu'indiqué précédemment, ceci s'avère particulièrement problématique en ce qui concerne l'application/administration par voie topique cutanée de ces acides nucléiques et dérivés. En effet, la couche cornée de l'épiderme de la peau agissant comme une barrière protectrice, la biodisponibilité cutanée et capacité de ces mêmes acides nucléiques et dérivés à pénétrer l'épiderme afin d'y exercer une action ciblée au niveau cellulaire, sont également susceptibles d'être affectées, voire afficher une diffusion très limitée lorsque des couches plus profondes de la peau sont visées (Zakrewsky M. et al., J. Control Release, 2015, vol.219, pp.445-456 et références citées). Pareillement à titre de « challenge » à surmonter en vue d'une utilisation optimale desdits acides nucléiques et dérivés en cosmétique et dermocosmétique, notamment dans le cadre d'une application par voie topique cutanée, figure leur comportement physico-chimique en solution. En effet, les PDRNs et les PNs ne se dissolvent pas facilement dans l'eau et nécessitent en général qu'ils soient soumis à un traitement thermique susceptible dès lors de dégrader leurs caractéristiques et qualités (Marques C. et al., Biomolecules, 2025, vol.15, 148). Quant aux susmentionnées nucléobases (également - plus simplement - dénommées « bases »), elles sont, d'une manière générale, elles aussi entravées dans leur potentiel applicatif par une faible solubilité dans l'eau (Ghoshdastidar D. et al., J. Phys. Chem. B., 2016, vol.120(3), pp.492-503). En matière de stratégies destinées à surmonter l'une quelconque de ces limitations d'emploi pour les acides nucléiques et dérivés, plusieurs concepts d'action générale ont été rapportés (Wang F. et al., Biosensors, 2022, vol.12, pp.1-35 et références citées). A titre illustratif, l'on peut citer, par exemple, leur intégration à différents types de nanomatériaux, tels que des nanoparticules à base de lipides ou des nanoparticules inorganiques, afin d'obtenir des performances supérieures dans les applications biomédicales visées et notamment dans le traitement des rides de la peau (Yeo S. et al., Pharmaceutics, 2021, vol.13, 33), ou encore citer leur association avec un panel d'agents hydrotropes porteurs d'une structure moléculaire amphiphile (demande de brevet US2014107059). L'état de la technique affiche également des modifications chimiques qui sont engagées sur des acides nucléiques naturels avec l'objectif d'améliorer leur comportement biologique, à l'image par exemple d'une classe de réactions chimiques biocompatibles (réunie sous le vocable expressif "chimie clic" ou "click chemistry" en anglais) qui cherche à leur "accrocher" par conjugaison des molécules bioactives et fonctionnelles spécifiques pour aboutir au final - dixit les auteurs dudit article - à des "hybrides nucléosidiques" (Perrone D. et al., Molecules, 2021, vol.26, 3100). Par ailleurs et en raison de son affinité pour les composés hétéroaromatiques, l'arginine serait ainsi en capacité d'accroître la solubilité de toutes les nucléobases testées dès lors que ces nucléobases sont électriquement neutres (Hirano A., Arch. Biochem. Biophys., 2010, vol.497(1-2), pp.90-96). Enfin, il est souligné l'approche plus lointaine visant à amplifier la solubilité de substrats dits faiblement solubles dans l'eau (ces derniers primo-illustrés dans l'étude par la nucléobase "adénine") par, et avec la détermination des constantes d'association apparente (Ka), la formation et l'obtention d'entités "complexe" résultant d'une association entre ledit substrat faiblement soluble dans l'eau et une sélection de ligands appropriés tels que des vitamines hydrosolubles et des acides aminés (Chen A.X. et al., Pharm. Res., 1994, vol.111(3), pp.398-401). D'autres entités, qualifiées de "complexes nucléo-peptidiques" et utilisées comme activateur de la mélanogénèse cutanée, s'affichent également dans l'état de la technique avec une séquence d'au moins trois acides aminés liés chimiquement à des acides puriques ou leurs dérivés xanthines (demande de brevet FR2753707 A1).

En tout état de cause, malgré cette mise à disposition de solutions diverses destinées à améliorer l'administration cutanée des acides nucléiques et dérivés, il est important de souligner que d'autres études font état à leur encontre, a contrario, de bénéfices limités voire nuls (Lee K.W.A. et al., Int. J. Mol. Sc., 2024, vol.25, 8224). Dans le même temps, subsiste le besoin de poursuivre toujours plus loin les recherches visant à établir des conditions d'utilisation et d'efficacité les plus optimales possibles pour une exploitation plus universelle des acides nucléiques et dérivés en cosmétique et dermocosmétique, notamment le fait d'évaluer, tel pour les PDRNs précités, leur fonction et efficacité dans des bioactivités/indications éclectiques (Nguyen T.H. et al., Int. J. Biol. Macromolecules, 2024, vol.282(3), 137051). Ainsi, la présente invention s'inscrit sans réserve dans un tel défi et besoin de concevoir et mettre à disposition [en particulier sur le marché des actifs cosmétiques et/ou dermocosmétiques catégorisés "acides nucléiques et dérivés"] un nouvel ingrédient répondant à tout ou partie des besoins susvisés et visant à atteindre tout ou partie des objectifs suivants :
i) répondre aux limites d'utilisation précitées autour des acides nucléiques et dérivés : stabilité, biodisponibilité et solubilité ;
ii) maximiser le potentiel biologique des acides nucléiques et de leurs dérivés, notamment pour prévenir et/ou lutter contre les désordres cosmétiques et dermocosmétiques susceptibles d'affecter aussi bien la physiologie de la peau du corps que celle, plus spécifique, du cuir chevelu ;
iii) afficher, de manière optionnelle (mains néanmoins avantageuse), une composante supplémentaire aux prérequis i) et ii), à savoir exposer une naturalité totale pour ledit ingrédient, en substance présenter un indice de naturalité de 100% au sens de la norme internationale ISO 16128 [texte visant à harmoniser et encadrer les pratiques des acteurs de l'industrie cosmétique autour du "naturel", notamment fournir des lignes directrices relatives aux définitions et aux critères applicables aux ingrédients et produits cosmétiques naturels et biologiques, en particulier constituer une méthode de détermination des indices "Naturel", "d'Origine Naturelle", "Biologique" et "d'Origine Biologique" applicables aux ingrédients cosmétiques - Numéro de référence : ISO 16128-2:2017(F)].

A propos de ce besoin (optionnel) iii) supra, son origine émane en effet d'une forte tendance et demande actuellement observées chez les formulateurs de produits cosmétiques, à savoir que, dans un monde heureusement de plus en plus soucieux des enjeux environnementaux et de la durabilité, l'industrie des cosmétiques évolue elle aussi en cherchant à intégrer graduellement des pratiques plus responsables et respectueuses de la planète. De ce fait, des concepts-clés tels que "naturalité", "économie circulaire", "éco-conception", "cosmétiques verts", ont émergé ces dernières années à l'instar de ceux rapportés par le livre blanc « *Economie circulaire et secteur cosmétique - 120 bonnes pratiques des entreprises de la cosmétique* » édité par la Fédération des Entreprises de la Beauté (FEBEA), syndicat professionnel français des fabricants de produits cosmétiques. En substance, ce livre blanc aborde les défis et les opportunités qui se présentent désormais pour le secteur cosmétique face à la nécessité de réduire l'impact environnemental en matière de production de produits cosmétiques. Il met ainsi en lumière des solutions concrètes pour encourager la réduction des déchets, l'optimisation des ressources et la réutilisation des matériaux, tout en veillant à maintenir la qualité et la sécurité du produit. A travers des recommandations détaillées, ce recueil de bonnes pratiques souligne la nécessité d'adopter une démarche plus responsable tout au long du cycle de vie des produits, depuis leur conception jusqu'à leur fin de vie. D'une manière générale, ce livre blanc invite l'ensemble des acteurs du secteur cosmétique à repenser notoirement leur mode de production, notamment en prônant une économie circulaire et une écologie dite "industrielle" avec principalement une minimisation des déchets, une maximisation dans l'utilisation des ressources, la mise en place de systèmes de recyclage efficaces ou encore d'écosystèmes durables inter-entreprises à l'image du (sic) « *Déchets pour les uns, matières premières pour les autres* » relevé sur la Toile. Placés en début de chaine d'approvisionnement du produit, les fournisseurs d'ingrédients cosmétiques sont d'autant plus confrontés à ce défi d'innover dans le contexte des quatre concepts-clés susmentionnés que le besoin aujourd'hui est grandissant voire immédiat pour des alternatives - aux ingrédients conventionnels d'origine fossile - qualifiées de, durables, efficaces et respectueuses de l'environnement et de la biodiversité (Goyal N., et al., Env. Sc. Pollution Res., 2023, vol.30, pp.25148-25169). La demanderesse et ses inventeurs ont donc eu également pour ambition de chercher à innover en considérant un tel constat.

### Exposé de l'invention

La présente invention a donc été développée dans le contexte de recherche et cahier des charges - ambitieux - tels qu'indiqués supra, impliquant la conception et la réalisation d'un panel d'essais visant à mettre au point et développer des modèles d'évaluation biologique, tant à visée cutanée que capillaire, susceptibles de démontrer l'efficacité et la mise en valeur des ingrédients testés. Ainsi, au terme d'un long travail de recherche, de surcroît contre toute attente eu égard à la connaissance admise que l'acide ortho-silicique Si(OH)₄ (ou H₄SiO₄), forme naturelle de silicium assimilable chez l'Homme, affiche désavantageusement une faible concentration dans l'eau (< 2 mM soit 56 mg Si/I) à cause d'une très grande tendance à s'auto-condenser dès que les teneurs en silicium s'élèvent, en conséquence à produire défavorablement des formes polymérisées peu, ou plus difficilement, absorbables par l'organisme et donc potentiellement inertes ou peu actives biologiquement (Jurkić L.M. et al., Nutr. Meta., 2013, vol.10, pp.1-12), la demanderesse est parvenue à concevoir, dans le respect de ratio et conditions maîtrisés, un nouveau complexe moléculaire (également dénommé complexe « supramoléculaire ») combinant en interaction (chimie supramoléculaire), d'une part, une famille limitée de nucléobases et dérivés avec, d'autre part, une forme (de préférence naturelle) de silicium soluble, biodisponible, biologiquement assimilable, qui plus est [conformément au prérequis optionnel objet du point susnommé iii)] avantageusement issu initialement d'un sous-produit (déchet) généré par l'industrie céréalière, et avec de surcroît au final un comportement général large et avantageux en cosmétique et dermocosmétique [conformément au prérequis objet du point susnommé ii)]. Ainsi, l'invention a pour objet un complexe (moléculaire) formé entre :
a) une biomolécule azotée hétérocyclique sélectionnée parmi les nucléobases puriques et/ou leurs alcaloïdes méthylxanthines dérivés, libres ou sous forme **N-**glycosylée; et
b) un acide silicique constitué de l'acide ortho-silicique et/ou d'au moins l'un de ses oligomères hydrosolubles;
ledit complexe étant formé via l'établissement d'au moins une liaison chimique faible, préférablement d'au moins une liaison hydrogène, entre au moins un groupement hydroxyle dudit acide silicique b) et ladite biomolécule azotée hétérocyclique a), de préférence au niveau d'au moins un des atomes d'azote de ladite biomolécule azotée hétérocyclique a) ; le ratio molaire entre a) et b) étant compris entre environ 0,5 (par exemple 0,5) et environ 15 (par exemple 15).

Selon un mode de réalisation, le complexe selon l'invention est formé via l'établissement de liaisons faibles, de préférence de liaisons hydrogène, entre les groupements hydroxyle dudit acide silicique b) et les atomes d'azote de ladite biomolécule azotée hétérocyclique a).

En effet, après un grand nombre d'essais menés tant au niveau du ratio entre les éléments constitutifs dudit complexe qu'au niveau des conditions opératoires, le choix de la demanderesse s'est en effet arrêté sur une telle nouvelle entité chimique et association intermoléculaire, sous forme de "complexe" tel que défini par les règles de nomenclature de l'Union Internationale de Chimie Pure et Appliquée ("UICPA" en français, ou "IUPAC" en anglais pour "International Union of Pure and Applied Chemistry"), en raison du comportement général avantageux et prometteur que ledit complexe selon l'invention a affiché à travers un faisceau d'effets techniques bénéfiques, notamment pour l'un desdits effets, la supériorité expérimentale que ledit complexe selon l'invention a affichée en comparaison de chacun des éléments constitutifs pris isolément (à comprendre par "non-engagé dans un même état de complexe "), ce faisceau d'effets techniques bénéfiques étant précisément illustré par :
- sa capacité à limiter significativement la sécrétion de lipides (sébum) produits et accumulés dans et par les sébocytes [cf. test 1 ci-après] ;
- sa capacité à stimuler significativement la prolifération de cellules de la papille dermique du follicule pileux humain (plus connues sous l'abréviation "HFDPC"), ladite papille étant située à la base du follicule pileux et jouant de ce fait un rôle important dans la phase de croissance (anagène) du cheveu (réf.) [cf. test 2 ci-après] ;
- sa capacité à limiter significativement une hyper-prolifération de kératinocytes induite par un stress inflammatoire, suggérant ainsi un effet protecteur du complexe selon l'invention sur lesdits kératinocytes (cellules constitutives, et de la couche superficielle de la peau que représente l'épiderme, et des phanères tels les cheveux et les ongles) [cf. test 3 ci-après] ;
- enfin, sa capacité à améliorer très significativement, sur explants de peau et en application topique, la viscoélasticité de la peau, suggérant ainsi la capacité du complexe selon l'invention à restaurer le comportement viscoélastique d'une peau âgée [cf. test 4 ci-après].

Dans ledit test 2 ci-après, il est souligné que l'étude menée et le complexe selon l'invention ont permis d'afficher un comportement biologique pour celui-ci de manière beaucoup plus efficace - effet largement supérieur - que la première composante dudit complexe (adénine fonctionnalisée par une liaison N-osidique, en l'occurrence un groupement ribofuranose par l'intermédiaire d'une liaison β-N(9)-glycosidique), seul, ou la seconde composante dudit complexe (acide silicique), seul.

De surcroît, pour ledit test 2 ci-après, il est relevé, de manière totalement inattendue, un phénomène de synergie d'action pour un tel complexe selon l'invention [se présentant, dans le cas présent, sous forme de complexe formé entre l'adénine fonctionnalisée par une liaison N-osidique, en l'occurrence un groupement ribofuranose par l'intermédiaire d'une liaison β-N(9)-glycosidique, et l'acide silicique] ;
il est d'ailleurs légitimement formulé une telle synergie d'action :
- au regard d'un enseignement théorique apporté pour l'emploi d'une telle locution dans le domaine des produits et substances chimiques, en particulier le fait que (sic) *« il y a effet synergique lorsque l'effet combiné de deux produits chimiques est de beaucoup supérieur à la somme des effets de chaque produit pris individuellement. Par exemple : 2 + 2 >> 4 »* [ https://www.cchst.ca/oshanswers/chemicals/synergism.html ] ;
- à la lumière de l'expérimentation et des résultats obtenus dans le test 2 ci-après, puisque l'association des deux composants objets du complexe selon l'invention, permet d'obtenir un bien meilleur effet que celui obtenu, à des concentrations d'utilisation équivalentes, pour chacun d'entre eux pris individuellement.

Il est en outre légitimement formulé que ce mode d'action spécifique, avec ce phénomène objectivé de synergie d'action, permet de qualifier le comportement biologique du susdit complexe illustré comme "optimal" ou "doué de propriétés optimales" pour la croissance du cheveu et donc comme ingrédient actif destiné à favoriser la pousse/repousse du cheveu. Il est enfin légitimement formulé la formation et l'existence d'un état de "complexe" (moléculaire), eu égard, d'une part aux définitions de "complexe" et de "liaison hydrogène" établies par l'Union Internationale de Chimie Pure et Appliquée ("UICPA" en français, ou "IUPAC" en anglais), d'autre part et pour les nucléobases puriques et dérivés qui ont été spécifiquement sélectionnés, à leurs régions riches en électrons, marquées par la présence de doublets électroniques non-liants et d'atomes d'hydrogènes acides sur certains atomes d'azote, propices justement à l'établissement de telles interactions de type "liaison hydrogène" avec au moins un groupement hydroxyle de l'acide silicique. A la connaissance de la demanderesse, et bien qu'un même état de "complexe" et de liaisons faibles aient déjà été décrits pour une solution d'acide ortho-silicique stabilisée par des sels d'un dérivé d'acide γ-aminé non protéinogène, en l'occurrence la carnitine (demande de brevet WO2012/035364 A1), ou encore pour de l'acide ortho-silicique associé à un polypeptide (demande de brevet EP1092728 A2), la susdite famille de biomolécules azotées hétérocycliques a) - clairement distincte des susdits stabilisants carnitine et polypeptide - et le susdit acide silicique b) (acide ortho-silicique et/ou au moins l'un de ses oligomères hydrosolubles) n'ont jamais été associés sous forme de complexe moléculaire, que ce soit en vue d'une utilisation à des fins cosmétiques et/ou dermocosmétiques, ou encore vis-à-vis de désordres/altérations susceptibles de viser tant la physiologie de la peau du corps que celle, plus spécifique, du cuir chevelu. En outre, un quelconque phénomène de synergie d'action, entre la susdite famille de biomolécules azotées hétérocycliques a) sélectionnée parmi les nucléobases puriques et/ou leurs alcaloïdes méthylxanthines dérivés, et le susdit acide silicique b), à l'état de "complexe" ou non (simple association), n'a jamais été décrit ni même présagé dans l'état de la technique. De surcroît, conformément aux objectifs attachés au problème technique posé par la présente invention, le susdit complexe selon l'invention présente l'intérêt, dans un mode de réalisation avantageux de l'invention, d'afficher un total indice de naturalité, à savoir un indice de 100% d'origine naturelle au sens de la norme internationale ISO 16128. Selon un mode de réalisation particulier, l'invention concerne également un complexe (moléculaire) formé entre :
a) une biomolécule azotée hétérocyclique sélectionnée parmi les nucléobases puriques et/ou leurs alcaloïdes méthylxanthines dérivés, libres ou sous forme **N-**glycosylée; et
b) un acide silicique constitué de l'acide ortho-silicique et/ou d'au moins l'un de ses oligomères hydrosolubles;
ledit complexe étant formé via l'établissement d'au moins une liaison chimique faible, préférablement d'au moins une liaison hydrogène, entre au moins un groupement hydroxyle dudit acide silicique b) et ladite biomolécule azotée hétérocyclique a), de préférence au niveau d'au moins un des atomes d'azote de ladite biomolécule azotée hétérocyclique a). Selon un mode de réalisation de l'invention, ladite biomolécule azotée hétérocyclique est une nucléobase purique libre, préférablement l'adénine ou la guanine, avantageusement l'adénine.

Selon un autre mode de réalisation de l'invention, ladite biomolécule azotée hétérocyclique est une nucléobase purique N-glycosylée, de préférence associée au D-ribose. Avantageusement, ladite nucléobase purique N-glycosylée est choisie parmi la 9-β-D-ribofuranosyladénine et/ou la 9-β-D-ribofuranosylguanine, de préférence la 9-β-D-ribofuranosyladénine (en nomenclature systématique IUPAC).

Selon un autre mode de réalisation de l'invention, ladite biomolécule azotée hétérocyclique est un alcaloïde méthylxanthine dérivé d'une nucléobase purique, choisi parmi les 1,3,7-triméthylxanthine, 1,3-diméthylxanthine, 3,7-diméthylxanthine et/ou acide 1,3-diméthylxanthine-7-acétique, de préférence consistant en la 1,3,7-triméthylxanthine (en nomenclature systématique IUPAC).

Selon un mode de réalisation de l'invention, ledit acide silicique est l'acide ortho-silicique (H₄SiO₄).

Selon un mode de réalisation de l'invention, lesdits oligomères hydrosolubles de l'acide ortho-silicique sont l'acide di-silicique (H₆Si₂O₇), l'acide tri-silicique linéaire (H₈Si₃O₄₀) et l'acide tri-silicique cyclique (H₆Si₃O₉), de préférence l'acide di-silicique (H₆Si₂O₇).

Selon un mode de réalisation de l'invention, ledit acide silicique est constitué d'acide ortho-silicique et éventuellement d'au moins l'un de ses oligomères hydrosolubles.

Selon un mode de réalisation de l'invention, ledit acide silicique est constitué d'acide ortho-silicique et d'au moins l'un de ses oligomères hydrosolubles. Dans ce mode de réalisation, la proportion d'acide ortho-silicique est préférablement égale ou supérieure à 70%, de préférence égale ou supérieure à 90%, avantageusement de 100%.

Dans un mode de réalisation préféré de l'invention, d'une part ladite biomolécule azotée hétérocyclique a) est l'adénine ou la 9-β-D-ribofuranosyladénine, de préférence la 9-β-D-ribofuranosyladénine, d'autre part ledit acide silicique b) est sélectionné parmi l'acide ortho-silicique et l'acide di-silicique.

Dans un mode de réalisation préféré de l'invention, ledit acide silicique b) est issu d'une biomasse végétale contenant du silicium, choisie de préférence parmi les espèces graminées *Oryza sativa* (riz), *Panicum miliaceum* (millet), *Triticum spp.* (blé), *Zea mays* (maïs), *Hordeum vulgare* (orge), *Avena sativa* (avoine), *Secale cereale* (seigle), *Sorghum bicolor* (sorgho), *Triticum dicoccum* (épeautre), *Triticum turanicum* (blé Khorasan), *Eragrostis* tef (teff), *Digitaria exilis* (fonio), et les pseudo-céréales *Chenopodium quinoa* (quinoa), *Amaranthus spp.* (amarante), *Fagopyrum esculentum* (sarrasin), en particulier choisie parmi les espèces graminées *Oryza sativa* (riz) et *Panicum miliaceum* (millet), avantageusement l'espèce *Oryza sativa* (riz).

De manière préférée, ladite biomasse végétale contenant du silicium est un sous-produit de l'industrie céréalière, de préférence la balle de *Oryza sativa* (riz). A cet égard en effet, et notamment pour les besoins objets du point susnommé iii), la demanderesse s'est tournée vers une matière première issue du recyclage (l' "upcycling" en anglais), en l'occurrence ladite balle de *Oryza sativa* (riz), et qui n'est autre que l'enveloppe externe qui recouvre le grain de riz. Les raisons du choix primaire de ladite matière ont été doubles : d'une part, elle constitue clairement un sous-produit naturel du décorticage du riz. En effet, après l'extraction des grains, ces balles de riz sont le plus souvent considérées comme un déchet agricole, produit en grande masse avec l'activité très importante que représente l'industrie du riz pour la population mondiale. Heureusement depuis plus d'une décennie, ces balles de riz et ces volumes importants de déchets trouvent de nombreuses applications dans des domaines aussi variés que l'aviculture, le compostage, la combustion où elle est utilisée comme biomasse pour alimenter des réacteurs et produire ainsi de l'énergie thermique ou électrique, etc. (Moraes C.A.M. et al., Waste Management & Research, 2014, vol.32(11), pp.1034-1048). D'autre part, et d'intérêt pour la demanderesse dans le contexte de sa recherche et cahier des charges tels qu'indiqués supra, il est mis en exergue que ces balles de riz sont également riches en dioxyde de silicium (SiO2), appelé plus communément "silice de balle de riz", alternative ainsi durable aux sources traditionnelles de silice pour les industries en quête de responsabilité environnementale (Islam T. et al., Asian J. Chem. Sci., 2024, vol.14(2), pp.83-96). Il est enfin rapporté que cette silice de balle de riz, une fois l'objet d'un traitement thermique, est convertie en silice amorphe, forme précurseur et candidat particulièrement intéressant pour la délivrance, jusqu'à sa limite de solubilité, de l'acide ortho-silicique susmentionné (Ali M. et al., Int. J. Cur. Res. Rev., 2021, vol.13(24), pp.94-99). Avantageusement:
i) le ratio molaire entre ladite biomolécule azotée hétérocyclique a) et ledit acide silicique b) est compris entre environ 1 et environ 7 (par exemple entre 1 et 7), avantageusement entre environ 2 et environ 3 (par exemple entre 2 et 3), et/ou
ii) le pH de de la composition (sous forme liquide, avantageusement sous forme de solution) comprenant ledit complexe est compris entre environ 2,5 et environ 4,5 (par exemple entre 2,5 et 4,5), avantageusement entre environ 3 et environ 3,5 (par exemple entre 3 et 3,5), avantageusement d'environ 3 (par exemple 3);
de manière préférée, le complexe et la composition liquide le comprenant (avantageusement sous forme de solution) présentant les caractéristiques i) et ii) tel qu'illustré par son procédé de préparation [cf. test 5 ci-après].

L'invention a également trait à une composition, de préférence sous forme de solution, ladite composition comprenant (ou consistant essentiellement en) au moins un complexe selon l'invention, dans laquelle :
i) le ratio molaire entre ladite biomolécule azotée hétérocyclique a) et ledit acide silicique b) est compris entre environ 0,5 et environ 15 (par exemple entre 0,5 et 15), de préférence entre 1 et environ 7 (par exemple entre 1 et 7), avantageusement entre environ 2 et environ 3 (par exemple entre 2 et 3), et/ou
ii) le pH de de la composition (sous forme liquide, avantageusement sous forme de solution) comprenant ledit complexe est compris entre environ 2,5 et environ 4,5 (par exemple entre 2,5 et 4,5), préférablement entre environ 3 et environ 3,5 (par exemple entre 3 et 3,5), avantageusement d'environ 3 (par exemple 3);
de manière préférée ladite composition présentant les caractéristiques i) et ii).

À titre incident, et d'un point de vue sémantique, il convient de noter que l'expression « consistant essentiellement en », employée ci-dessus, présente la même signification que l'expression « constitué essentiellement de » ; ces deux formulations étant interchangeables. Selon un mode de réalisation particulier, l'invention concerne une composition, de préférence sous forme de solution, ladite composition comprenant (ou consistant essentiellement en) au moins un complexe formé entre :
a) une biomolécule azotée hétérocyclique sélectionnée parmi les nucléobases puriques et/ou leurs alcaloïdes méthylxanthines dérivés, libres ou sous forme N-glycosylée (tel que défini précédemment); et
b) un acide silicique constitué de l'acide ortho-silicique et/ou d'au moins l'un de ses oligomères hydrosolubles (tel que défini précédemment);
ledit complexe étant formé via l'établissement d'au moins une liaison chimique faible, préférablement d'au moins une liaison hydrogène, entre au moins un groupement hydroxyle dudit acide silicique b) et ladite biomolécule azotée hétérocyclique a), de préférence au niveau d'au moins un des atomes d'azote de ladite biomolécule azotée hétérocyclique a) ; de préférence, le pH de de ladite composition (sous forme liquide, avantageusement sous forme de solution) étant compris entre environ 2,5 et environ 4,5 (par exemple entre 2,5 et 4,5), préférablement entre environ 3 et environ 3,5 (par exemple entre 3 et 3,5), avantageusement d'environ 3 (par exemple 3).

L'invention a également trait à une composition cosmétique ou dermocosmétique, caractérisée en ce que ladite composition comprend (ou consiste essentiellement en), en association avec au moins un excipient cosmétiquement ou dermocosmétiquement acceptable, au moins un complexe ou une composition selon l'invention, de manière préférée ladite composition cosmétique ou dermocosmétique étant en outre sous une forme adaptée à une administration par voie topique cutanée et ledit au moins un excipient étant en outre physiologiquement acceptable avec la peau. Avantageusement, la quantité de ladite composition selon l'invention dans les compositions susmentionnées est comprise entre 1 % et 10 % en poids par rapport au poids total de ladite composition, de préférence entre 3 % et 7 % en poids par rapport au poids total de ladite composition. A titre indicatif et non limitatif, lorsque les susdites compositions à usage cosmétique ou dermocosmétique sont adaptées à une administration par voie topique cutanée, elles sont susceptibles de se présenter sous toutes les formes normalement utilisées pour une telle voie d'administration. Ainsi et de manière avantageuse, elles peuvent se présenter sous une forme liquide, avantageusement sous forme d'une solution (par exemple sous forme de solution aqueuse ou hydroalcoolique), éventuellement gélifiée, d'une dispersion du type lotion éventuellement biphasée, d'une émulsion huile-dans-eau ou eau-dans-huile, d'une suspension, d'une lotion, d'une crème, d'un gel aqueux ou hydroalcoolique, ou bien encore sous la forme d'une poudre (micronisée), d'une mousse, d'un sérum, d'une pâte, d'une suspension, d'une dispersion ou d'émulsions multiples pouvant être éventuellement des microémulsions ou des nanoémulsions. En terme d'adjuvants présents dans les compositions susvisées et physiologiquement compatibles avec la peau, l'on peut citer un composé choisi parmi les huiles, les cires, les élastomères de silicone, les tensioactifs, les co-tensioactifs, les solubilisants, les co-solubilisants, les épaississants et/ou gélifiants, les humectants, les émollients, les filtres solaires organiques ou inorganiques, les photostabilisants, les conservateurs à l'exception des conservateurs donneurs d'aldéhydes, les colorants, les charges abrasives, les nacres, les agents matifiants, les agents tenseurs, les agents séquestrants, les parfums, etc., et leurs mélanges. A titre indicatif et non limitatif, un tel adjuvant acceptable dans les domaines d'application visés par la présente invention, présent dans la formule dans une teneur de l'ordre de 0,01 à 20% en poids par rapport au poids total de la composition, préparation et autre complément susvisé(e), est notamment cité dans le dictionnaire "International Cosmetic Ingredient Dictionary and Handbook" publié par le "Personal Care Products Council (PCPC)" de l'Association cosmétique américaine. Enfin, ces mêmes compositions susvisées peuvent, dans certains modes de réalisations, comprendre, au moins un ingrédient actif additionnel, l'homme du métier veillant toutefois à ce que ces éventuels actifs additionnels, ainsi que leurs proportions, soient choisis de telle manière que les propriétés avantageuses reconnues à ces compositions susvisées ne soient pas, ou substantiellement pas, altérées par l'adjonction envisagée. De tels actifs additionnels peuvent être choisis, sans que cette liste ne soit limitative, parmi les agents dépigmentants, les agents photoprotecteurs, les agents stimulant la fonction barrière, les agents hydratants ou humectants, les agents desquamants, les agents apaisants, les agents exfoliants, les agents stimulant la prolifération cellulaire tels les fibroblastes et les kératinocytes, les agents déglycosylants, les agents stimulant la synthèse de collagène ou d'élastine ou prévenant leur dégradation, les agents stimulant la synthèse de glycosaminoglycanes ou de protéoglycanes ou prévenant leur dégradation, les agents antioxydants ou anti-radicalaires ou anti-pollution, les agents stimulant la lipolyse, les agents drainants ou détoxifiants, les agents anti-inflammatoires, les agents accélérateurs de pénétration, les agents desquamants, les agents apaisants et/ou anti-irritants, les agents astringents, les agents exfoliants, les agents agissant sur la microcirculation, etc., et leurs mélanges, un tel ingrédient actif additionnel étant présent dans la formule dans une teneur de l'ordre de 0,0001 à 20% en poids, de préférence de 0,01 à 5% en poids, par rapport au poids total de la composition susvisée. L'invention concerne également l'utilisation cosmétique ou dermocosmétique non-thérapeutique d'un complexe selon l'invention tel que défini supra, ou d'une composition cosmétique ou dermocosmétique telle que définie précédemment, pour :
a) corriger les états hyperséborrhéiques non pathologiques de la peau et/ou du cuir chevelu, de préférence du cuir chevelu, avantageusement pour corriger les irritations du cuir chevelu liées à des états hyperséborrhéiques non pathologiques du cuir chevelu ; et/ou
b) favoriser la pousse ou la repousse du cheveu ; et/ou
c) prévenir et/ou diminuer les signes cutanés du vieillissement, de préférence choisis parmi une peau présentant une altération de ses propriétés viscoélastiques et une peau présentant une altération dans la cohésion de ses tissus.

Tel qu'indiqué précédemment, l'utilisation cosmétique ou dermocosmétique non-thérapeutique susmentionnée vise la peau « saine » ou le cheveu « sain » - ou la/les zone(s) de peau saine - par opposition à une peau ou du cheveu « malade » - ou une/des zone(s) de peau malade - présentant une pathologie cutanée ou capillaire.

Dans le cas d'une administration topique cutanée, cette utilisation s'effectue sur une ou plusieurs zone(s) de peau « saine(s) » ou sur du cheveu « sain », c'est-à-dire une ou plusieurs zone(s) de peau, ou du cheveu, non affecté(s) par une pathologie cutanée ou capillaire.

Selon un mode de réalisation particulier, notamment dans le cas de voies d'administration différentes de l'administration topique cutanée, l'utilisation cosmétique ou dermocosmétique non-thérapeutique susmentionnée s'effectue chez un individu ne présentant pas de pathologie cutanée ou capillaire. Cet individu est qualifié, dans le contexte de la présente invention, de « sujet sain », par opposition à un « sujet malade » présentant une pathologie cutanée ou capillaire.

Un autre aspect de l'invention, distinct de l'aspect « *cosmétique* » ou « *dermocosmétique* » ci-avant, concerne également par ailleurs l'aspect « *dermatologique* ». A ce titre, l'invention a également pour objet un complexe (ou une composition le comprenant ou constitué essentiellement d'un tel complexe) formé entre :
a) une biomolécule azotée hétérocyclique sélectionnée parmi les nucléobases puriques et/ou leurs alcaloïdes méthylxanthines dérivés, libres ou sous forme **N-**glycosylée; et
b) un acide silicique constitué de l'acide ortho-silicique et/ou d'au moins l'un de ses oligomères hydrosolubles;

ledit complexe étant formé via l'établissement d'au moins une liaison chimique faible, préférablement d'au moins une liaison hydrogène, entre au moins un groupement hydroxyle dudit acide silicique b) et ladite biomolécule azotée hétérocyclique a), de préférence au niveau d'au moins un des atomes d'azote de ladite biomolécule azotée hétérocyclique a) ;
le ratio molaire entre a) et b) étant compris entre environ 0,5 et environ 15 (par exemple entre 0,5 et 15),
pour son utilisation dans le traitement de la séborrhée pathologique, de l'acné, de la rosacée et/ou de la dermatite atopique.

L'invention concerne également l'utilisation d'un acide silicique b) tel que défini précédemment pour :
i) favoriser la biodisponibilité d'une biomolécule azotée hétérocyclique a) tel que définie précédemment; et/ou
ii) accroître la solubilité d'une biomolécule azotée hétérocyclique biomolécule azotée hétérocyclique a) tel que définie précédemment.

### Définitions

### Définitions générales

Par « *acides nucléiques et dérivés* », il faut comprendre tant une macromolécule (polymère) organique constituée d'une succession de nucléotides que ses unités monomériques constitutives (ces dernières pouvant être libres ou fonctionnalisées par une liaison **N-**osidique).

Par « *acide ortho-silicique* », il faut comprendre la molécule de structure brute (H₄SiO₄).

Par « *au moins l'un de ses oligomères hydrosolubles* », il faut comprendre l'une ou plusieurs des formes oligomériques telles que figurant dans l'article rédigé par l'équipe japonaise "Igarashi M. et al, Nature communications, 2017, vol.8(140), pp.1-8".

Par « *complexe* » ou « *complexe moléculaire* », il faut comprendre une entité moléculaire formée par une association libre entre deux ou plusieurs entités moléculaires composantes (ioniques ou non chargées), ou les espèces chimiques correspondantes, la liaison entre les composants étant par ailleurs normalement plus faible que dans le cas d'une liaison covalente (Source: IUPAC, 1994, 66, 1077 - Glossary of terms used in physical organic chemistry - IUPAC - Recommendations 1994 - on page 1098).

Par « *liaison hydrogène* », il faut comprendre une interaction attractive entre un atome d'hydrogène d'une molécule ou d'un fragment moléculaire X-H dans lequel X est plus électronégatif que H, et un atome ou un groupe d'atomes d'une autre molécule. Une liaison hydrogène typique peut être représentée par X-H•••Y-Z, où les trois points désignent la liaison et dans laquelle X-H représente le donneur de liaison hydrogène, l'accepteur étant une région riche en électrons tel qu'un doublet non liant sur Y. La preuve de la formation de liaison hydrogène peut être expérimentale ou théorique, ou une combinaison des deux (Henry M., Interference International Review of Science, 2015, et références citées).

Par « *en nomenclature systématique IUPAC* », il faut comprendre une dénomination selon un système standardisé des composés chimiques basé sur l'application des recommandations les plus strictes établies par l'Union Internationale de Chimie Pure et Appliquée ("UICPA" en français, ou "IUPAC" en anglais pour "International Union of Pure and Applied Chemistry"), recommandations qui visent à s'assurer qu'un nom correspond à une et une seule structure moléculaire.

Par « *synergie d'action* », il faut comprendre qu'il y ait effet synergique lorsque l'effet combiné de deux produits chimiques est supérieur à la somme des effets de chaque produit pris individuellement. Par exemple : 2 + 2 >> 4. [https://www.cchst.ca/oshanswers/chemicals/synergism.html ].

Par « *dans le respect de ratio et conditions maîtrisés* », il faut comprendre un rapport/ratio molaire biomolécule azotée hétérocyclique limitée à celle des nucléobases puriques ou l'un de ses alcaloïdes méthylxanthines dérivés a) / acide silicique b), établi après un criblage comparatif d'essais multiples visant à définir un ratio entre ces deux entités, susceptible de conférer au complexe selon l'invention un comportement biologique sur la peau qui puisse être le plus favorable possible, ainsi que l'identification de conditions opératoires qui permettent de maintenir simultanément une solubilisation des entités biomolécule azotée hétérocyclique a) et acide silicique b).

Par « *états hyperséborrhéiques non pathologiques de la peau et*/*ou du cuir chevelu* » , il faut comprendre une sécrétion excessive de sébum qui donne à la peau ou au cuir chevelu un aspect gras, luisant, voire huileux, sans être associé à une pathologie dermatologique.

Par « *leurs alcaloïdes méthylxanthines dérivés* », comme cela est compris de l'homme du métier , l'on entend des composés de type xanthine, méthylés et principalement issus du métabolisme secondaire des plantes et de leurs nucléobases puriques (Monteiro J.P. et al., Molecules, 2016, vol.21, 974 ; Ashihara H. et al., Plant Nucleotide Metabolism - Biosynthesis, Degradation, and Alkaloid Formation, 2020, pp.213-229).

### Description détaillée

La description détaillée ci-après a pour but d'exposer l'invention de manière suffisamment claire et complète, notamment à l'aide d'exemples, mais ne doit en aucun cas être regardée comme limitant l'étendue de la protection aux modes de réalisation particuliers et aux exemples présentés ci-après.

### Exemples

Exemple 1 : il est mentionné ci-après trois illustrations de formulation de composition selon l'invention, à visée cosmétique ou dermocosmétique, toutes trois contenant un complexe selon l'invention précitée :

**Tableau 1**

| **Formule A (crème)** | |
|---|---|
| Complexe selon l'invention [acide ortho-silicique/ adénine] | 5% |
| Triglycérides capriques/capryliques | 5% |
| Hémisqualane | 3% |
| Alcools C14-22 (et) glucosides d'alkyle C12-20 | 1,7% |
| Polyacrylate 6-crosspolymère | 0,5% |
| Glycérine | 2% |
| 4-hydroxyacétophénone | 0,5% |
| Caprylate de glycéryle | 0,75% |
| Eau | qsp 100% |

**Tableau 2**

| **Formule B (sérum cuir chevelu)** | |
|---|---|
| Complexe selon l'invention [acide ortho-silicique/ 9-β-D-ribofuranosyladénine] | 5% |
| 4-hydroxyacétophénone | 0,5% |
| Erythritol | 3% |
| Chlorure de triméthyl-[2-(2-méthylprop-2-énoyloxy)éthyl]azanium | 0,5% |
| 2,4-dihydroxy-N-(3-hydroxypropyl)-3,3-diméthylbutanamide | 0,3% |
| Alcool dénaturé | 5% |
| Caprylate de glycéryle | 0,75% |
| Eau | qsp 100% |

**Tableau 3**

| **Formule C (lotion capillaire)** | |
|---|---|
| Complexe selon l'invention [acide ortho-silicique/ 1,3,7-triméthylxanthine] | 4% |
| Benzoate de sodium | 0,7% |
| Glycérine (végétale) | 3% |
| Gomme Xanthane | 0,05% |
| Butylène glycol | 2% |
| Phénoxyethanol | 0,5% |
| Eau | qsp 100% |

Par ailleurs à propos de la susdite formule B, il est souligné que son exploitation dans une étude *in vivo* menée sur l'Homme (test clinique, réalisé sous couvert de confidentialité) a donné lieu à des résultats préliminaires très favorables (VS. placebo) sur le cuir chevelu ("scalp"), tant au niveau de son état séborrhéique (diminution de la production de sébum) que celui de son hydratation (augmentation).

D'autres d'études *in vivo* menées sur l'homme (tests cliniques, réalisés sous couvert de confidentialité) et avec la susdite formule B sont également en cours aux fins d'illustrer l'exploitation de la présente invention, précisément avec un regard sur les paramètres suivants (VS. placebo) :
- diminution du nombre de cheveux qui tombent au brossage
- diminution du nombre de cheveux arrachés suite à une traction
- augmentation de la densité
- augmentation du nombre de cheveux en phase anagène
- diminution du nombre de cheveux en phase télogène.

### Exemple 2 : l'invention est illustrée ci-après, à titre purement indicatif par les tests suivants évoqués plus haut dans la description de l'invention (tests 1 à 5)

### Test 1 : mise en évidence de la capacité du complexe selon l'invention à limiter la sécrétion de lipides produits et accumulés dans et par les sébocytes

Expérimentalement, le test est réalisé sur un modèle de lignée cellulaire de sébocytes humains immortalisés, modifié pour exprimer un récepteur fonctionnel à androgène (réf. Bioalternatives "SEBO66662AR" dans le rapport d'étude de Qima Life Sciences No SD230106). Précisément, ces sébocytes "SEBO6662" sont ensemencés dans une plaque à 96 puits et cultivés pendant 24 heures dans un milieu de culture. Ce milieu est ensuite remplacé par le milieu d'essai contenant, ou non (cas du contrôle), la référence "cérulénine" (inhibiteur naturel de la biosynthèse d'acide gras et du métabolisme des stéroïdes) ou l'ingrédient actif à tester. Après une nouvelle incubation de 4 heures, un mélange stimulant lipogène (mélange de vitamine C, vitamine D3, insuline, calcium et dihydrotestostérone "DHT") est alors ajouté et les cellules sont à nouveau incubées pendant 7 jours. A J+3, la moitié du milieu est retiré et les traitements (y compris le mélange lipogène) sont renouvelés. Des conditions de contrôle non stimulées sont réalisées en parallèle. A J+7, les cellules sont rincées, fixées et perméabilisées. Les gouttelettes lipidiques contenues dans les cellules sont ensuite marquées à l'aide d'une sonde lipidique fluorescente spécifique (Bodipy^{®}) puis soumises à une analyse d'images à l'aide d'un logiciel (INCell Analyzer^{™} 2200 - GE Healthcare). Dix photos sont prises par puits (objectif x20). Le marquage est quantifié par la mesure de l'intensité de fluorescence normalisée par rapport au nombre total de cellules (% moyen). Toutes les conditions expérimentales sont réalisées en triplicate (n=3). Les résultats, notamment exprimés en pourcentage de lipides accumulés, sont rassemblés dans le tableau 4 ci-après. Ils ont été obtenus pour les différentes configurations suivantes :
- cas 1, à savoir :
   . le "contrôle", à savoir sans l'ajout d'un ingrédient actif;
- cas 2, à savoir :
   . l'agent inhibiteur de référence (cérulénine) ;
- cas 3, à savoir :
   . le complexe selon l'invention, en solution [lot n° E18127], associant (liaisons hydrogènes), d'une part, le composé "9-β-D-ribofuranosyladénine" (au titre de la biomolécule azotée hétérocyclique a)) et, d'autre part, l'acide ortho-silicique et/ou au moins l'un de ses oligomères hydrosolubles (au titre de l'acide silicique b), l'acide ortho-silicique étant l'entité silicique de départ), dans le ratio molaire (a/b) de 2,28.

**Tableau 4**

| **Composé (concentration)** | **quantité (%) de lipides** | **inhibition sur formation (%) de lipides** |
|---|---|---|
| Cas 1 [contrôle] | 100 | N/A |
| Cas 2 [référence] (10 µM) | 54 | - 46 |
| Cas 3 [selon l'invention] (0,3 %) | 78 | - 22 |

Les résultats soulignent, pour le complexe selon l'invention, une capacité à inhiber significativement la formation de gouttelettes lipidiques induite par un mélange lipogène contenant des androgènes, soit une capacité à réduire la synthèse et le stockage de lipides dans les sébocytes, d'intérêt notamment pour corriger les états hyperséborrhéiques non pathologiques de la peau et/ou du cuir chevelu.

### Test 2 : mise en évidence de la capacité du complexe selon l'invention à stimuler significativement la prolifération de cellules de la papille dermique du follicule pileux humain

Expérimentalement, le test est réalisé sur des cellules humaines de la papille dermique de follicule humain (abréviation "HFDPC" pour "Human Follicle Dermal Papilla Cells") obtenues à partir d'un lifting temporal d'une patiente caucasienne âgée de 66 ans (fournisseur Promocell : référence C-12071 - lot 485Z013). Les cellules sont ensuite cultivées à 37°C et 5% CO₂ dans un milieu de culture ("Human Follicle Dermal Papilla Cell Growth Medium + supplement Mix" - fournisseur Promocell : référence C-26501).

A J0, ces HFDPC sont ensemencées dans des plaques 24 puits à raison de 1.10⁴ cellules/cm². A J+1, les HFDPC sont alors traitées, dans 500 µl de milieu de culture "HFDPC Growth Medium", en présence des composés à tester. A J+2, les effets des traitements sur la prolifération cellulaire sont évalués à l'aide d'un test fréquemment utilisé dans les cellules HFDPC, à savoir le « test MTT » ou "test au bromure de 3-(4,5-diméthylthiazol-2-yl)-2,5-diphenyltétrazolium (MTT)". Le milieu de culture est en effet remplacé par 500 µL d'une solution à 0,5 g/L de MTT. Puis après 3h d'incubation, le formazan généré est dissout dans 500 µL de DMSO. Les absorbances sont alors mesurées à 570 nm à l'aide du "spectrophotomètre Biotech EPOCH2 (Agilent)". La prolifération cellulaire est finalement exprimée en % de DO₅₇₀ₙₘ par rapport à la DO₅₇₀ₙₘ moyenne des témoins non-traités. Les résultats, rassemblant les valeurs moyennes obtenues à partir de trois expérimentations indépendantes, sont présentés dans le tableau 5 ci-après. Ils ont été obtenus pour les différents configurations suivantes :
- cas 1, à savoir :
   . le "contrôle", à savoir sans ajout d'un ingrédient actif;
- cas 2, à savoir :
   . le composé "9-β-D-ribofuranosyladénine" [lot n° E18427], testé seul ;
- cas 3, à savoir :
   . l'acide ortho-silicique et/ou au moins l'un de ses oligomères hydrosolubles [lot n° E18428], testé(s) seul(s) ;
- cas 4, à savoir :
   . le complexe selon l'invention, en solution [lot n° 5148.23], associant (liaisons hydrogènes), d'une part, le composé "9-β-D-ribofuranosyladénine" (au titre de la biomolécule azotée hétérocyclique a)) (cas 3) et, d'autre part, l'acide ortho-silicique et/ou au moins l'un de ses oligomères hydrosolubles (au titre de l'acide silicique b), l'acide ortho-silicique étant l'entité silicique de départ) (cas 4), dans le ratio molaire (a/b) de 2,28.

**Tableau 5**

| **Composé (concentration)** | **prolifération (%) cellulaire** | **majoration (%) prolifération des HFDPC** |
|---|---|---|
| Cas 1 [contrôle] | 100 | N/A |
| Cas 2 (0,25 %) | 99 | - 1 % |
| Cas 3 (0,25 %) | 105 | +5% |
| Cas 4 [selon l'invention] (0,25 %) | 113 | + 13 % |

Les résultats soulignent, pour le complexe selon l'invention, en comparaison de ses deux éléments constitutifs pris isolément, une capacité très largement supérieure, de surcroît même par un phénomène objectivé de synergie d'action, à stimuler la prolifération de cellules de la papille dermique du follicule pileux humain, d'intérêt notamment pour favoriser la pousse ou la repousse du cheveu.

### Test 3 : mise en évidence de la capacité du complexe selon l'invention à limiter significativement une hyper-prolifération de kératinocytes induite par un stress inflammatoire

Expérimentalement, le test est réalisé sur une lignée cellulaire de kératinocytes immortalisés "HaCaT" (fournisseur : ATCC), maintenue par repiquage dans du milieu de culture complet "DMEM" (contenant 1 g/l de glucose supplémenté avec 10% de sérum de veau fœtal) en atmosphère humide à 37 °C et 5 % de CO₂. A J0, les cellules HaCaT sont ensemencées dans des plaques 24 puits à raison de 2.10⁴ cellules/cm². A J+1 et J+2, les cellules sont traitées dans du milieu de culture complet "DMEM" contenant 0,5 % de sérum de veau fœtal, avec les composés à tester et en présence d'un stress inflammatoire généré par l'ajout (15 ng/ml) de lipopolysaccharides (LPS) bactériens responsables d'une réaction d'hyper-prolifération des kératinocytes ("LPS stress"). Après deux fois 24 h de traitement, les cellules sont détachées, récupérées et comptées avec la pipette de comptage "Scepter Millipore", et les valeurs sont présentées comme la moyenne du nombre de cellules. Les résultats, rassemblant les valeurs moyennes obtenues à partir de trois expérimentations indépendantes, sont présentés dans le tableau 6 ci-après. Ils ont été obtenus pour les différents configurations suivantes :
- cas 1, à savoir :
   . le "contrôle", à savoir sans ajout d'un ingrédient actif et sans LPS-stress ;
- cas 2, à savoir :

   . le "contrôle LPS", à savoir sans ajout d'un ingrédient actif et avec LPS-stress ;
- cas 3, à savoir (avec LPS-stress) :
   . le complexe, en solution [lot n° 5120.22], associant les composés "méthylsilanetriol", "(3-hydroxypropoxy)-(méthyl)silanediol", et "9-β-D-ribofuranosyladénine", dans le ratio molaire de 1, les composés tels que le méthylsilanetriol ou le (3-hydroxypropoxy)-(méthyl)silanediol étant des formes de « silicium organique » dont dispose la demanderesse, et qui sont connues pour afficher un comportement dans l'eau significativement supérieur à celui de l'acide ortho-silicique, ainsi que d'excellentes propriétés en termes d'assimilation et de stabilité ;
- cas 4, à savoir (avec LPS-stress) :
   . le complexe selon l'invention, en solution [lot n° E18127], associant (liaisons hydrogènes), d'une part, le composé "9-β-D-ribofuranosyladénine" (au titre de la biomolécule azotée hétérocyclique a)) et, d'autre part, l'acide ortho-silicique et/ou au moins l'un de ses oligomères hydrosolubles (au titre de l'acide silicique b), l'acide ortho-silicique étant l'entité silicique de départ), dans le ratio molaire (a/b) de 2,28.

**Tableau 6**

| **Composé (concentration)** | **quantité de cellules/cm²** | **stimulation (%) de la prolifération** |
|---|---|---|
| Cas 1 [contrôle] | 143 388 | N/A |
| Cas 2 | 171 813 | +20% |
| Cas 3 (0,5 %) | 152 025 | +6% |
| Cas 4 [selon l'invention] (0,5 %) | 130 438 | -9% |

Les résultats soulignent, pour le complexe selon l'invention, une capacité à réduire significativement l'hyper-prolifération de kératinocytes induite par un stress inflammatoire.

### Test 4 : mise en évidence de la capacité du complexe selon l'invention à améliorer la viscoélasticité de la peau

Expérimentalement, l'essai est réalisé sur des explants de peaux issues d'abdominoplasties de 55 ans (référence Biomeca : 23031PA) et 61 ans (référence : 23032PA), conservés dans un milieu de survie "DMEM-antibiotiques". Plusieurs "punchs", à comprendre par des disques de peau comprenant l'épiderme et le derme, de même taille (1,20 cm de diamètre), sont placés sur un insert à l'interface air/liquide dans un milieu de survie (Gibco DMEM + cocktail d'antibiotiques, référence Biomeca: 31966021). Les prélèvements cutanés sont alors placés à température et humidité contrôlées pendant l'expérience (humidité relative : > 93% à 37°C). Les composés à tester sont appliqués par voie topique sur toute la surface des punchs (application de 100µl au centre des punchs), 2 fois par jour pendant 1 jour et ½ selon un protocole défini (rapport d'étude Biomeca, réf. DEV-2023-0495). Les acquisitions en "Microscopie à Force Atomique (AFM)", consistant en l'acquisition de force-volume (FV) au niveau de la peau dans un environnement air, sont directement réalisés sur les explants de peaux et obtenues à l'aide d'un microscope de force atomique (Bioscope Resolve - Bruker) auquel est greffé un microscope à épifluorescence (Leica DMi8). Une telle combinaison permet également d'acquérir des images corrélatives entre les acquisitions mécaniques et fluorescentes. Les acquisitions de force-volume (FV) obtenues correspondent alors à une multitude de points de mesure, dont il est extrait finalement les propriétés viscoélastiques, exprimées en "viscoélasticité (UA)". Les résultats, rassemblant les valeurs moyennes obtenues à partir de quatre expérimentations indépendantes, sont présentés dans le tableau 7 ci-après. Ils ont été obtenus pour les différents configurations suivantes :
- cas 1, à savoir :
   . le "contrôle", à savoir sans l'ajout d'un ingrédient actif;
- cas 2, à savoir :
   . le complexe, en solution [lot n° 5120], associant les composés "méthylsilanetriol", "(3-hydroxypropoxy)-(méthyl)silanediol", et "9-β-D-ribofuranosyladénine" (cas 2), dans le ratio molaire de 1, les composés tels que le méthylsilanetriol ou le (3-hydroxypropoxy)-(méthyl)silanediol étant des formes de « silicium organique » dont dispose la demanderesse, et qui sont connues pour afficher un comportement dans l'eau significativement supérieur à celui de l'acide ortho-silicique, ainsi que d'excellentes propriétés en termes d'assimilation et de stabilité ;
- cas 3, à savoir :
   . le complexe selon l'invention, en solution [lot n° 18127], associant (liaisons hydrogènes), d'une part, le composé "9-β-D-ribofuranosyladénine" (au titre de la biomolécule azotée hétérocyclique a)) et, d'autre part, l'acide ortho-silicique et/ou au moins l'un de ses oligomères hydrosolubles (au titre de l'acide silicique b), l'acide ortho-silicique étant l'entité silicique de départ), dans le ratio molaire (a/b) de 2,28.

**Tableau 7**

| **Composé (concentration)** | **visco-élasticité globale (UA)** | **majoration (%) de la visco-élasticité (UA)** |
|---|---|---|
| Cas 1 [contrôle] | 1 | N/A |
| Cas 2 (5 %) | 1,16 | + 16% |
| Cas 3 [selon l'invention] | 1,89 | + 89% |
| (5 %) | | |

Les résultats soulignent, pour le complexe selon l'invention, une capacité à améliorer, de surcroît de manière très significative, les propriétés viscoélastiques de la peau, d'intérêt notamment pour améliorer l'hydratation et la fonction "barrière" de la peau ainsi que pour corriger la formation de rides et un relâchement de la peau.

### Test 5 : procédé de préparation d'un complexe selon l'invention formé entre, d'une part, la 9-β-D-ribofuranosyladénine (au titre de la biomolécule azotée hétérocyclique a)),et d'autre part, un acide silicique constitué de l'acide ortho-silicique et/ou au moins l'un de ses oligomères hydrosolubles (au titre de l'acide silicique b), l'acide ortho-silicique étant l'entité silicique de départ)

Afin d'obtenir 100 g de complexe selon l'invention, la 9-β-D-ribofuranosyladénine (8,7 g ; 32,5 mmoles) est tout d'abord solubilisée dans un mélange composé d'eau déminéralisée (40 g) et de 1,3-dihydroxypropane (50 g). Le pH de la solution est alors acidifié avec un acide inorganique et/ou organique, jusqu'à un pH égal à 3. Sous agitation mécanique et à température ambiante ensuite, il est ajouté 0,44 g (14,2 mmoles) d'une solution concentrée de silicates (solution à 90 g/kg en silicium) obtenue à partir de balles de riz (sous-produit de l'industrie céréalière contenant 9% de silicium) à la précédente solution susmentionnée de 9-β-D-ribofuranosyladénine, tout en veillant à maintenir le pH du mélange entre 3 et 3,5 (à nouveau à l'aide d'un acide inorganique et/ou organique). Après un complément avec de l'eau déminéralisée (jusqu'à qsp 100) puis une étape de filtration, l'on obtient une solution (complexe) limpide et incolore.

## Revendications

1. Complexe formé entre :
a) une biomolécule azotée hétérocyclique sélectionnée parmi les nucléobases puriques et/ou leurs alcaloïdes méthylxanthines dérivés, libres ou sous forme **N-**glycosylée; et
b) un acide silicique constitué de l'acide ortho-silicique et/ou d'au moins l'un de ses oligomères hydrosolubles;
ledit complexe étant formé via l'établissement d'au moins une liaison chimique faible, préférablement d'au moins une liaison hydrogène, entre au moins un groupement hydroxyle dudit acide silicique b) et ladite biomolécule azotée hétérocyclique a), de préférence au niveau d'au moins un des atomes d'azote de ladite biomolécule azotée hétérocyclique a) ;
le ratio molaire entre a) et b) étant compris entre environ 0,5 et environ 15.

2. Complexe selon la revendication 1, dans lequel ladite biomolécule azotée hétérocyclique est une nucléobase purique libre, préférablement l'adénine ou la guanine, avantageusement l'adénine.

3. Complexe selon la revendication 1, dans lequel ladite biomolécule azotée hétérocyclique est une nucléobase purique N-glycosylée, de préférence associée au D-ribose.

4. Complexe selon la revendication 3, dans lequel ladite nucléobase purique N-glycosylée est choisie parmi la 9-β-D-ribofuranosyladénine et/ou la 9-β-D-ribofuranosylguanine, de préférence la 9-β-D-ribofuranosyladénine.

5. Complexe selon la revendication 1, dans lequel ladite biomolécule azotée hétérocyclique est un alcaloïde méthylxanthine dérivé d'une nucléobase purique, choisi parmi les 1,3,7-triméthylxanthine, 1,3-diméthylxanthine, 3,7-diméthylxanthine et/ou acide 1,3-diméthylxanthine-7-acétique, de préférence consistant en la 1,3,7-triméthylxanthine.

6. Complexe selon l'une des revendications 1 à 5, dans lequel ledit acide silicique est l'acide ortho-silicique.

7. Complexe selon l'une des revendications 1 à 6, dans lequel lesdits oligomères hydrosolubles sont l'acide di-silicique, l'acide tri-silicique linéaire et l'acide tri-silicique cyclique, de préférence l'acide di-silicique.

8. Complexe selon l'une des revendications 1 à 7, dans lequel ledit acide silicique b) est issu d'une biomasse végétale contenant du silicium, de préférence choisie parmi les espèces graminées *Oryza sativa* (riz), *Panicum miliaceum* (millet), *Triticum spp.* (blé), *Zea mays* (maïs), *Hordeum vulgare* (orge), *Avena sativa* (avoine), *Secale cereale* (seigle), *Sorghum bicolor* (sorgho), *Triticum dicoccum* (épeautre), *Triticum turanicum* (blé Khorasan), *Eragrostis* tef (teff), *Digitaria exilis* (fonio), et les pseudo-céréales *Chenopodium quinoa* (quinoa), *Amaranthus spp.* (amarante), *Fagopyrum esculentum* (sarrasin), en particulier choisie parmi les espèces graminées *Oryza sativa* (riz) et *Panicum miliaceum* (millet), avantageusement l'espèce *Oryza sativa* (riz) ;
de manière préférée, ladite biomasse végétale contenant du silicium étant un sous-produit de l'industrie céréalière, de préférence la balle de *Oryza sativa.*

9. Composition, de préférence sous forme de solution, ladite composition comprenant au moins un complexe selon l'une des revendications 1 à 8, dans laquelle :
i) le ratio molaire entre ladite biomolécule azotée hétérocyclique a) et ledit acide silicique b) est compris entre environ 0,5 et environ 15, préférablement entre environ 1 et environ 7, avantageusement entre environ 2 et environ 3, et/ou
ii) le pH de de ladite composition est compris entre environ 2,5 et environ 4,5, préférablement entre environ 3 et environ 3,5, avantageusement d'environ 3 ;
de manière préférée ladite composition présentant les caractéristiques i) et ii).

10. Composition cosmétique ou dermocosmétique, **caractérisée en ce que** ladite composition comprend, en association avec au moins un excipient cosmétiquement ou dermocosmétiquement acceptable, au moins un complexe selon l'une des revendications 1 à 8 ou une composition selon la revendication 9, de préférence ladite composition cosmétique ou dermocosmétique étant sous forme de solution ;
de manière préférée ladite composition cosmétique ou dermocosmétique étant en outre sous une forme adaptée à une administration par voie topique cutanée et ledit au moins un excipient étant en outre physiologiquement acceptable avec la peau.

11. Composition cosmétique ou dermocosmétique selon la revendication précédente, **caractérisée en ce que** ladite composition comprend en outre au moins un ingrédient actif additionnel choisi parmi les agents dépigmentants, les agents photoprotecteurs, les agents stimulant la fonction barrière, les agents hydratants ou humectants, les agents desquamants, les agents apaisants, les agents exfoliants, les agents stimulant la prolifération cellulaires tels les fibroblastes et les kératinocytes, les agents déglycosylants, les agents stimulant la synthèse de collagène ou d'élastine ou prévenant leur dégradation, les agents stimulant la synthèse de glycosaminoglycanes ou de protéoglycanes ou prévenant leur dégradation, les agents antioxydants ou anti-radicalaires ou anti-pollution, les agents stimulant la lipolyse, les agents drainants ou détoxifiants, les agents anti-inflammatoires, les agents accélérateurs de pénétration et/ou anti-irritants, les agents astringents, les agents agissant sur la microcirculation, et leurs mélanges.

12. Utilisation cosmétique ou dermocosmétique non-thérapeutique d'un complexe selon l'une des revendications 1 à 8 ou d'une composition selon l'une des revendications 9 à **11,** pour :
i) corriger les états hyperséborrhéiques non pathologiques de la peau et/ou du cuir chevelu, de préférence du cuir chevelu, avantageusement pour corriger les irritations du cuir chevelu liées à des états hyperséborrhéiques non pathologiques du cuir chevelu ; et/ou
ii) favoriser la pousse ou la repousse du cheveu ; et/ou
iii) prévenir et/ou diminuer les signes cutanés du vieillissement, de préférence choisis parmi une peau présentant une altération de ses propriétés viscoélastiques et une peau présentant une altération dans la cohésion de ses tissus.

13. Complexe selon l'une des revendications 1 à 8 ou composition selon la revendication 9, pour son utilisation dans le traitement de la séborrhée pathologique, de l'acné, de la rosacée et/ou de la dermatite atopique.

14. Utilisation d'un acide silicique constitué de l'acide ortho-silicique et/ou d'au moins l'un de ses oligomères hydrosolubles pour :
i) favoriser la biodisponibilité d'une biomolécule azotée hétérocyclique sélectionnée parmi les nucléobases puriques et/ou leurs alcaloïdes méthylxanthines dérivés, libres ou sous forme N-glycosylée ; et/ou
ii) accroître la solubilité d'une biomolécule azotée hétérocyclique sélectionnée parmi les nucléobases puriques et/ou leurs alcaloïdes méthylxanthines dérivés, libres ou sous forme N-glycosylée.
